# EUROPEAN PATENT SPECIFICATION

(11) **EP 1 898 724 B1**
(45) Date of publication and mention of the grant of the patent: **25.08.2010**
(21) Application number: 06757833.6
(22) Date of filing: 30.06.2006
(51) Int. Cl.: A23L 1/29, A23L 1/305, A61P 3/04

(54) **INFANT NUTRITION WITH HYDROLISED PROTEINS**
BABYNAHRUNG MIT HYDROLISIERTEN PROTEINEN
NUTRITION DU NOURRISSON AVEC DES PROTEINES HYDROLYSEES

(30) Priority: 01.07.2005 EP 05106002
(43) Date of publication of application: 19.03.2008
(73) Proprietor: N.V. NUTRICIA, 2712 HM Zoetermeer (NL)
(72) Inventor: BEERMANN, Christopher, 61267 Neu-Anspach (DE); VAN LAERE, Katrien, Maria, Josefa, NL-6666 WS Heteren (NL); VAN BEEK, Eline, Marleen, NL-6705 DB Wageningen (NL); BOEHM, Günther, 61209 Echzell (DE)
(74) Representative: Swinkels, Bart Willem
(86) International application number: PCT/NL2006/050158
(87) International publication number: WO 2007/004878

(56) References cited:
- EP-A- 0 629 350
- EP-A- 0 631 731
- WO-A-98/36734
- WO-A-03/005836
- WO-A-2004/069265
- WO-A-2004/112509
- WO-A-2005/039319
- US-A1- 2003 008 810
- US-B1- 6 384 087
- FLEISCHER MICHAELSEN, K.; HOPPE,C.; MOLGAARD, C.: "Effect of early protein intake on linear growth velocity and development of adiposity" MONATSSCHR KINDERHEILKUND, vol. 151, 2003, pages S78-S83, XP002389613

## Description

### FIELD OF THE INVENTION

The present invention relates to the field of infant nutrition, especially nutritionally complete formula and the use of such infant nutrition in the prevention and/or treatment of childhood obesity.

### BACKGROUND OF THE INVENTION

Childhood obesity is an increasing problem in developed countries. For instance, in the United States in the year 2000 about 15 % of the children (up to age 11) were considered to be obese, whereas in 1980 this was 7 %. Also in Europe an increase of childhood obesity is observed.

Children with obesity are very likely to have obesity persist into adulthood. Childhood obesity is associated with elevated blood pressure and lipids, and increased risk of diseases, such as asthma, type 2 diabetes and cardiovascular diseases at a later stage of life. Furthermore, being overweight is considered by the majority of the Western population as unattractive. Causes of childhood obesity include lack of regular physical exercise, sedentary behaviour, eating habits, socio-economic factors and genetic factors. Also early nutrition plays an important role in the prevention of childhood obesity; Armstrong & Reilly (2002, Lancet 359:2003-4) observed that feeding human milk lowers the risk of childhood obesity.

Breast-feeding is the preferred method of feeding infants. However, there are circumstances that make breast-feeding impossible or less desirable. In those cases infant formula and follow-on formula are a good alternative. The composition of modem infant or follow-on formulas is adapted in such a way that it meets the special nutritionally requirements of the fast growing and developing infant. A formula fed infant is (almost) completely dependent on these formula for its food and water. Therefore, the normal remedies for obesity, e.g. limiting the amount of ingested calories, inhibiting uptake of nutrients, increase of satiety, decrease of appetite, or an increase in thermogenesis, are not feasible, since the strict nutritional needs of the infant are imperilled. W02004069265 describes the use of peptides derived from whey protein hydrolysate as active ingredient in a medicament or as food ingredient for elevating the cholecystokinin level in the blood, and for preventing or treatment of overweight and/or obesity, in an animal, including human, in need thereof.

WO03074129 describes a nutritional supplement composition having therapeutically effective amounts of milk minerals including calcium, a protein source including casein fragment 106-169, and enzyme-inhibiting peptides for the treatment of body weight conditions. The nutritional supplement composition is administered in amounts effective for limiting weight gain and/or enhancing weight loss, as well as promoting overall good health, in the treatment of body weight conditions, including overweight and obesity.

WO03005836 describes dietary products for infant, child and adult nutrition which possess adequate levels and ratios of medium chain fatty acids and omega-polyunsaturated fatty acids. Consumption of these dietary products can contribute to the prevention of obesity in developing individuals and can contribute to a reduction in body fat mass in individuals who are trying to loose weight or reduce body fat mass (e.g., obese individuals).

EP629350 describes the use cow milk protein hydrolysates in infant nutrition to induce cow milk protein tolerance and prophylaxis or treatment of type 1 diabetes mellitus in children susceptible to such a disease.

WO2005039319 describes symbiotic infant compositions comprising non-digestible oligosaccharides and whey protein hydrolysates for the prevention or treatment of immune disorders.

EP0631731 describes the use of a milk protein partial hydrolysate in infant formula having reduced antigenicity.

US2003008810 describes a dry nutritional composition for inducing satiety by CKK release and producing weight loss comprising caseinmacropeptides, glycomacropeptides or hydrolysed glycomacropeptides.

### SUMMARY OF THE INVENTION

Feeding infants with infant formula results in a higher post-prandial insulin response than when the infants receive human milk, while blood glucose levels are not lowered. Because breast-feeding is believed to prevent childhood obesity, increased levels of both insulin and glucose as a result of feeding infant formula compared to feeding human milk are undesirable. Increased levels of both insulin and glucose indicate a form of insulin resistance in formula fed infants, which is believed contribute to the development of childhood obesity.

The inventors surprisingly found that the administration of a composition wherein the protein comprises hydrolysed casein and hydrolysed whey resulted in reduced post-prandial levels of both insulin and glucose compared to the administration of a composition comprising intact casein and intact whey protein. The post-prandial insulin response and post-prandial blood glucose levels observed after administration of hydrolysed casein and hydrolysed whey were comparable and closer to those observed when feeding human milk.

Hence, the present inventors have found that a combination of hydrolysed casein and hydrolysed whey proteins can be advantageously used in a method for the treatment and/or prevention of childhood obesity. The present invention can also suitably be used in a method for the treatment and/or prevention of secondary disorders in children suffering from childhood obesity, particularly one or more secondary disorders selected from the group consisting of (type 2) diabetes, insulin resistance, cardiovascular diseases, hypertension, asthma, sleep apnoea, orthopaedic complications (especially of the legs and hips bones) and arthritis.

In a further aspect the present invention provides a nutritional composition for use in the treatment and/prevention of childhood obesity, said composition comprising hydrolysed casein, hydrolysed whey and a non-digestible, fermentable carbohydrate, wherein said composition is for administration to an infant.

### DETAILED DESCRIPTION OF THE INTENTION

In one aspect, the present invention provides the use of fat, digestible carbohydrates and protein, wherein the protein comprises at least 25 wt. % peptides with a chain length of 2 to 30 amino acids based on dry weight of protein, for the manufacture of a nutritional composition for use in the treatment and/or prevention of childhood obesity.

In a further aspect, the present invention provides a nutritional composition for use in the treatment and/or prevention of childhood obesity, said nutritional composition comprising fat, digestible carbohydrates and protein, wherein the protein comprises at least 25 wt. % peptides with a chain length of 2 to 30 amino acids based on dry weight of protein, wherein said composition is for administration to an infant.

In still another aspect, the abovementioned nutritional composition can suitably be used in the treatment and/or prevention of type 2 diabetes, asthma, arthritis, hypertension, sleep apnoea, cardiovascular diseases, and/or orthopaedic disorders in a subject suffering from childhood obesity.

In yet another aspect, the invention provides a nutritional composition comprising 35 to 60 en. % fat, 25 to 75 en. % digestible carbohydrates, 5 to 16 en. % protein, and a non-digestible fermentable carbohydrate selected from the group consisting of polyfructose and galactooligosaccharides, wherein the protein comprises: at least 25 wt. % peptides with a chain length of 2 to 30 amino acids based on dry weight of protein; at least 50 wt. % mammalian milk derived proteins, based on weight of protein; casein and whey in a weight ratio casein:whey of 10:90 to 90: 10; hydrolysed whey protein and hydrolysed casein; and less than 15 wt.% free amino acids based on the weight of protein.

The term "childhood obesity", as used in the present invention, refers to the disease wherein children suffer from obesity or overweight. Both infants and children can suffer from childhood obesity. Particularly, children with a gender specific BMI-for age above the 85^{th} percentile, or even above the 95^{th} percentile are suffering from childhood obesity. BMI (body mass index) is an anthropometric measure, defined as weight in kilograms divided by the square of length in metres. Tables with gender specific BMI-for age are publicly available for instance at the US National Center for Health Statistics. If a child has a gender specific BMI-for age above the 85^{th} percentile or even 95^{th} percentile, this means that 85 % or even 95 % of the population consisting of children with the same age and sex have a lower BMI.

### Protein

The present composition contains protein comprising at least 25 wt. % peptides with a chain length of 2 to 30 amino acids based on dry weight of protein. The amount of peptides with a chain length of 2 to 30 amino acids can for example be determined as described by de Freitas et al, 1993, J. Agric. Food Chem 41:1432-1438.

The present composition preferably contains at least 50 wt. % protein derived from non-human milk based on total protein, more preferably at least 90 wt. %. Preferably the present composition contains at least 50 wt. % cow milk derived protein based on total protein, more preferably at least 90 wt. %. Preferably the present composition comprises protein derived from acid whey and/or sweet whey with a reduced concentration of glycomacropeptide. Typically, glycomacropeptide (GMP) with a molecular weight of 8000 daltons is a casein-derived whey protein containing amino acid residues 106-169 of kappa-casein, and is released from kappa-casein by the proteolytic action of rennin

(chymosin). GMP is glycosylated. The glycosylation part attached to the peptide backbone of the GMP molecule preferably comprises 7 to 8 % N acetylneuramic acid, commonly known as sialic acid. In one embodiment the present composition comprises whey protein derived from cow's milk with an increased concentration of glycomacropeptide Preferably the present composition comprises protein derived from β-casein and/or α-lactalbumin. Preferably the present composition contains hydrolysed whey protein and hydrolysed casein. Casein and whey proteins are preferably present in a weight ratio casein:whey of 10:90 to 90:10, more preferably 20:80 to 80:20. This is an optimal range, since the amino acid composition of bovine casein is more similar to the amino acid composition found in human milk protein and whey protein is easier to digest and found in greater ratios in human milk.

The present composition preferably includes both casein hydrolysate and whey protein hydrolysate because the amino acid composition of bovine casein is more similar to the amino acid composition found in human milk protein and whey protein is easier to digest and found in greater ratios in human milk.

The present composition preferably comprises rice protein. Rice protein and its partial hydrolysates are hypo-allergenic, well digestible and have an intermediately balanced amino acid composition. In order improve the amino acid balance the rice protein preferably is combined with essential amino acids such as lysine. In a preferred embodiment the rice protein is combined with legume protein, more preferably pea or bean protein.

The present composition preferably contains 5 to 16 en. % protein, most preferably 8.0 to 12.0 en. % protein. En. % is short for energy percentage and represents the relative amount each constituent contributes to the total caloric value of the preparation. The energy in the composition is derived from digestible carbohydrates, fat and protein. The term protein as used in the present invention refers to the sum of proteins, peptides and free amino acids.

The composition preferably contains 1.0 to 6.0 g, more preferably 1.0 to 2.5 g of protein per 100 ml of the ready to feed composition. The composition comparably preferably comprises at least 7.0 wt. %, more preferably at least 8.0 wt. % protein based on dry weight of the composition. Preferably, the composition comprises at most 40 wt. %, more preferable at most 20 wt. % of protein based on dry weight of the total composition The wt.% protein based on dry weight of the composition is calculated according to Kjeldahl percentage, N*6.25, in which N is the amount of nitrogen measured.

The composition preferably contains at least 50 wt. %, preferably at least 80 wt. %, most preferably about 100 wt. % of a protein hydrolysate, based on total weight of the protein.

The composition preferably contains less than 10 g free amino acids per 100 g protein, more preferably less than 7 g. A relatively low amino acid content results in a low osmolarity and thus prevents disturbances of the gastrointestinal tract/digestive system such as diarrhoea. A low content of free amino acids is of further importance for reducing the bitter taste; free amino acids give the formula a bitter taste. Furthermore, free amino acids are absorbed worse in the intestinal tract compared to peptides. Therefore the amount of free amino acids in the present composition is preferably limited.

The present composition contains at least 25 wt. % peptides with a chain length of 2 to 30 amino acids based on total protein %, preferably at least 40 wt. %, most preferably at least 50 wt. %. Preferably, the present composition contains at least 25 wt. % peptides with a chain length of 2 to 15 amino acids based on total protein, preferably at least 40 wt. %, most preferably at least 50 wt. %. Preferably, at least 0.5 wt. %, more preferably at least 1 wt. %, most preferably, at least 5 wt. % of the protein consists of peptides with a chain length between 15 and 30 amino acids. Preferably, the present composition contains between 0.5 and 8 wt. % peptides with a chain length of more than 30 amino acids based on total protein, more preferably between 0.7 to 7 wt. %, and even more preferably between 0.75 and 5 wt. %. The presence of small peptides (with a chain length of 2 to 15 amino acids) improves the digestibility of the proteins, and thereby of the entire formula. Also, the presence of peptides of between 15 and 30 amino acids will allow the digestive system of the infant to get used to larger and/or intact peptides. Preferably, at least 80 wt.%, more preferably at least 95 wt.% of the protein component has a chain length above 4, more preferably above 9 amino acids. In other words, preferably less than 20 wt.% more preferably less than 5 wt.% of the protein component consists of free amino acids and peptides with a chain length up to and including 4, more preferably up to and including 9 amino acids, for reasons of lower osmolarity, improved taste and improved digestibility of the product.

The present composition preferably contains a protein with a degree of hydrolysis of the protein of 5 to 25 %, more preferably of 7.5 to 21 %, most preferably of 10 to 20 %. The degree of hydrolysis is defined as the percentage of peptide bonds which have been broken down by enzymatic hydrolysis, with 100 % being the total potential peptide bonds present. Proteins with the abovementioned This degree of hydrolysis provides sufficient peptides with a chain length of 2 to 30

### Digestible carbohydrates

The present composition preferably contains digestible carbohydrates, preferably lactose. The composition preferably contains 25 to 75 en.% digestible carbohydrates, more preferably 40 to 55 en.%. The present composition preferably contains 6 to 19 g digestible carbohydrates per 100 ml, more preferably 6 to 10 g per 100 ml ready-to-feed liquid. Per 100 g dry weight the composition comparably contains 40 to 75 g digestible carbohydrates.

Because it is important that the insulin response and glycaemic index is low, preferably at least 35 wt.%, more preferably at least 50 wt.%, even more preferably at least 75 wt.%, most preferably at least 95 wt.% of the digestible carbohydrate in the present composition is lactose.

### Fat

The present composition contains fats. The amount of saturated fatty acids is preferably below 58 wt.% based on total fatty acids, more preferably below 45 wt.%. The concentration of monounsaturated fatty acids preferably ranges from 17 to 60 % based on weight of total fatty acids. The concentration of polyunsaturated fatty acids in the present composition is preferably between 11 and 36 % based on weight of total fatty acids. The fats are essential to the growth of the infant, while on the other hand the fat may contribute to occurrence of childhood obesity. The present fat mixture is believed to optimally prevent childhood obesity in the present protein containing composition.

The essential fatty acids linolenic acid (LA; an omega 6 fatty acid) and α-linolenic acid (ALA; an omega 3 fatty acid), should be present in sufficient amounts and in a balanced ratio, since LA and ALA deficiency and imbalance are correlated with conditions such as insulin resistance and obesity. The composition therefore preferably contains 0.3 to 1.5 g LA per 100 ml of the ready-to-feed liquid composition, and at least 50 mg ALA per 100 ml. The present composition preferably contains 1.8 to 12.0 wt. % LA based on dry weight of the composition, and at least 0.30 wt. % ALA based on dry weight of the composition. The weight ratio LA/ALA is preferably between 5 and 15. Preferably the present composition contains long chain polyunsaturated fatty acids (LC-PUFA), more preferably eicosapentaenoic acid (EPA) and/or docosahexaenoic acid (DHA). Both DHA and EPA improve the insulin sensitivity and are therefore advantageously included in the present composition. A fat composition with the properties as described above is believed to act synergistically with the hydrolysed protein on the prevention and/or treatment of childhood obesity.

The composition preferably contains 2.1 to 6.5 g fat per 100 ml when in ready-to-feed liquid form. Based on dry weight, the composition preferably contains 12.5 to 30 wt. % fat. The present composition preferably contains 35 to 60 en. % fat, more preferably 39 to 50 en.% fat.

### Non-digestible, fermentable carbohydrates

Non-digestible carbohydrates are carbohydrates that enter the human colon intact after oral ingestion. The term "fermentable" as used herein refers to the capability to undergo (anaerobic) breakdown and conversion by micro-organisms in the lower part of the gastro-intestinal tract (e.g. colon) to smaller molecules, in particular short chain fatty acids and lactate. The fermentability may be determined by the method described in Am. J. Clin. Nutr. 53, 1418-1424 (1991).

Non-digestible, fermentable carbohydrates (NDFC) have a blood glucose tempering effect, because they delay gastric emptying and shorten the small intestinal transit time. This effect may be caused via the short-chain fatty acids produced from the oligosaccharides in the colon via the so called ileo-colonic brake, which refers to the inhibition of gastric emptying by nutrients reaching the ileo-colonic junction. Short-chain fatty acids may also shorten ileal emptying. Therefore NDFC and hydrolysed proteins are believed to synergistically prevent and/or treat childhood obesity.

According to a preferred embodiment the composition contains one or more non-digestible, fermentable carbohydrates. The composition preferably contains 0.1 - 1.5 g, preferably 0.2 to 1.0 g NDFC per 100 ml ready-to-feed liquid composition. The composition preferably contains at least 0.5 wt.% NDFC based on total dry weight of the composition, more preferably at least 1.2 wt.%, most preferably at least 2.5 wt. %. Preferably, the composition comprises at most 15, more preferably at most 12, most preferably at most 8 wt. % NDFC based on dry weight of the composition. Preferably, the composition contains at least one NDFC selected from the group consisting of polyfructose, fructo-oligosaccharides, galacto-oligosaccharides, partially hydrolysed galactomannan, galacturonic acid comprising oligosaccharides and polydextrin oligosaccharides resistant to digestion in the human intestinal tract. Preferably the present composition contains a mixture of galacto-oligosaccharides. In an especially preferred embodiment the composition contains polyfructose. Polyfructose reduces total cholesterol, LDL cholesterol, VLDL and trygliceride levels in obese subjects. In an even further preferred embodiment the composition contains a mixture of transgalacto-oligosaccharides and polyfructose. This mixture synergistically produces the highest amounts of short chain fatty acids. In a preferred embodiment the weight ratio trans-galacto-oligosaccharides/polyfructose is between 99/1 and 1/99, more preferably between 98/2 and 1/9, even more preferably between 98/2 and 1/1.

Polyfructose is a polysaccharide carbohydrate comprising a chain of β linked fructose units with a degree of polymerisation or average degree of polymerisation of 10 or more. The degree of polymerisation or average degree of polymerisation of polyfructose is prefferabl;y below 100. Polyfructose includes inulin, levan and/or a mixed type of polyfructan. An especially preferred polyfructose is inulin. Polyfructose suitable for use in the compositions is also already commercially available, e.g. Raftiline®HP (Orafti).

Trans-galacto-oligosaccharides (TOS) are galacto-oligosaccharides in which the majority of the galactose units (preferably at least 90 % galactose units based on total monosaccharide units, more preferably at least 95 %) are linked by β-bonds. Most preferably TOS is used in which the majority of the galactose units (at least 50 %, preferably at least 90 %) are linked by β (1,4) and/or β (1,6) glycosidic bonds. Such a TOS is for example that found in Vivinal®GOS (Borculo Domo Ingredients, Zwolle, Netherlands).

### Probiotics

In a preferred embodiment the composition further contains probiotics. Probiotics are live micro-organisms which reach the colon alive upon oral ingestion and have a beneficial effect. Preferred probiotics belong to the genus *Bifidobacteria* and/or *Lactobacilli.* Proteolytic enzymes of the probiotics further hydrolyse the protein source, in the gastro-intestinal tract, thereby providing a synergetic effect. Furthermore, probiotics improve the intestinal flora, thereby improving the formation of short chain fatty acids, which have a blood glucose tempering effect. It is therefore especially preferred that the composition of the invention comprises both probiotics and NDFC, which may serve as a substrate for the probiotics. Preferably, probiotics are present in the composition in a concentration of 10³ to 10¹¹, more preferably 10⁵ to 10¹⁰, most preferably 10⁶ to 10⁹ colony forming units per g dry weight of the composition. Most preferably the present composition comprises *Bifidobacterium breve* and/or *Lactobacillus paracasei.* The present inventors found that the growth of these bacteria in impaired in the intestine of formula fed infants compared to breast fed infants.

### Polyamines

The present invention also provides the use of polyamines for treating and/or preventing childhood obesity and/or to treat and/or prevent type 2 diabetes, asthma, arthritis, hypertension, sleep apnoea, cardiovascular diseases, and/or orthopaedic disorders in a subject suffering from childhood obesity. In a preferred embodiment the present composition comprises polyamine. In a further preferred embodiment the composition comprises polyamines and the present protein hydrolysate. Preferably the polyamine is selected from the group consisting of cadaverine, putrescine, spermine and spermidine. Most preferably the composition contains spermidine and/or spermine. The present composition preferably contains 0.5 to 200 µg polyamines per 100 ml of the ready to use product, more preferably 2 to 40 µg. Preferably, the composition comprises at least two or more selected from the group consisting of cadaverine, spermine, spermidine and putrescine. Preferably the composition comprises about 10 to 90 wt. % of spermine, 10 to 90 wt. % of spermidine, 0 to 90 wt. % of putrescine and 0 to 20 wt. % of cadaverine based on weight of the polymanines. Preferably the present composition contains lactic acid bacteria, yeast extract and/ or pancreas extract as a source of polyamines. The polyamines play an important role in prevention and/or treatment of childhood obesity, since they modify postprandial kinetics, and effect the digestibility of proteins.

### Liquid composition

The present composition is preferably for administration in liquid form. In order to meet the caloric requirements, the composition preferably contains 50 to 200 kcal/100 ml, more preferably 60 to 90 kcal/100 ml. The nutritional composition preferably comprises 5 to 16 en.% protein, 35 to 60 en.% fat, and 25 to 75 en.% carbohydrates. The osmolarity of the present composition is typically between 150 and 420 mOsmol/l, preferably 260 to 320 mOsmol/l. The low osmolarity aims to reduce the gastrointestinal stress, e.g. reduce the incidence of diarrhoea, particularly in infants.

Preferably the composition is in a liquid form, with a viscosity below 35 cps. Suitably, the composition is in a powdered from, which can be reconstituted with water to from a liquid, or in a liquid concentrate form, which should be diluted with water.

### Daily dosages

When the composition is a liquid form, the preferred volume for administration on a daily basis is in the range of about 80 to 2500 ml, more preferably about 450 to 1000 ml per day, which is a suitable amount for an infant.

### Treatment

The present composition can advantageously be used in the treatment and/or prevention of childhood obesity. The present composition can also advantageously be used in the treatment and/or prevention of type 2 diabetes, hypertension, cardiovascular diseases, arthritis, asthma, sleep apnoea, and/or orthopaedic complications (especially of the leg and hip bones) in infants suffering from childhood obesity.

The present composition is preferably for oral administration to the infant. The composition is particularly useful for providing nutrition to an infant and/or stimulating the growth of an infant. The composition is particularly suitable for use in the prevention of childhood obesity and/or secondary disorders related thereto. Hence the composition is advantageously for administration to an infant of 0-24 months, preferably to an infant of 0-18 months.

### LEGENDS TO THE FIGURES:

Figure 1: Post-prandial blood glucose levels in rats fed a composition containing lactose, intact whey and intact casein (●), a composition containing lactose and hydrolysed whey and hydrolysed casein (○) or human milk (▲).
Figure 2: Post-prandial insulin levels in rats fed a composition containing lactose, intact whey and intact casein (●), a composition containing lactose and hydrolysed whey and hydrolysed casein (○) or human milk (▲).

### EXAMPLES

### Example 1

### Protein preparations

Intact whey protein, Deminal 90, was obtained from Borculo Domo Ingredients. Intact casein protein, was obtained from skimmed milk powder, with a casein/whey ratio of 4. Whey and casein protein were mixed in such a way that a ratio of 40 wt.% casein protein and 60 wt.% whey protein was obtained.

Hydrolysed whey protein was obtained by hydrolysis of an acid whey preparation as described in example 1-4 of WO0141581. The degree of hydrolysis was 15 %. Hydrolysed casein was commercially obtained as LacProdan DI-2038 (Arla Foods). The two preparations were combined at a ratio of 40 wt % hydrolysed casein and 60 wt % hydrolysed whey.

### Animals:

20 adult male Wistar rats (aged 10 weeks at the start of the experiment) were housed individually. The animals had *ad libitum* access to water and food (Standard Rat Chow, Harlan). The animals received a permanent canula in the jugular vein during surgery under isoflurane/N₂O/O₂ anaesthesia, to enable stress-free repeated blood sampling. All animal experiments were approved by the Animal Experimental Committee (DEC-Consult).

### Treatment:

After a 4 h fasting period, 10 animals were fed 2 ml of a composition. Three different compositions were tested in a cross-over design (experiments separated by one week).
   1 human breast milk (containing 11 mg protein, 70 mg lactose and 45 mg fat per ml.)
   2 17 mg whey/casein protein and 86 mg lactose per ml.
   3 17 mg hydrolyzed whey and hydrolyzed casein protein and 86 mg lactose per ml. Subsequently, blood samples (200 µl) were collected in heparinised chilled tubes at t=0, 5, 10, 15, 30, 60, 90, and 120 minutes after feeding. Subsequently, plasma was separated after centrifugation (10 min, 5000 rpm) and stored at-20 °C until analysis.

### Measurement of insulin:

Plasma insulin was measured by radioimmunoassay (RIA, Linco) according to the kit protocol with the following adjustment: all assay volumes were reduced four times.

### Measurement of glucose:

Plasma glucose was measured with an oxidase-peroxidase method in 96-wells format (Roche Diagnostics, #1448668).

### Area under the curve, peak time, maximal peak height:

The AUC was determined using the linear trapezoid rule. Area under the curve for glucose and insulin was calculated per animal, during the early peak (t=0-30 min) and under the entire curve measured (t=0-120 min). Negative values (when a response reaches levels below basal) were subtracted. Per animal the time and level of glucose and insulin peak was determined (maximum plasma concentrations of all measured time points).

### Results:

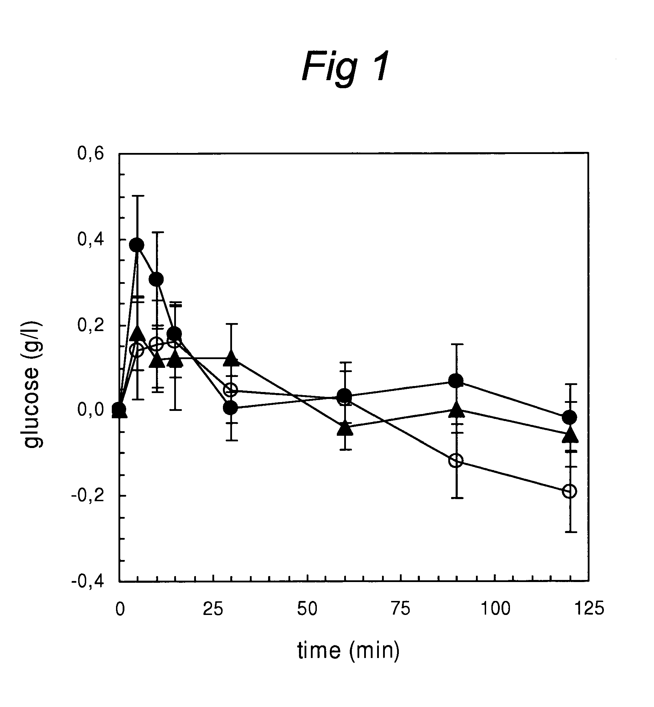

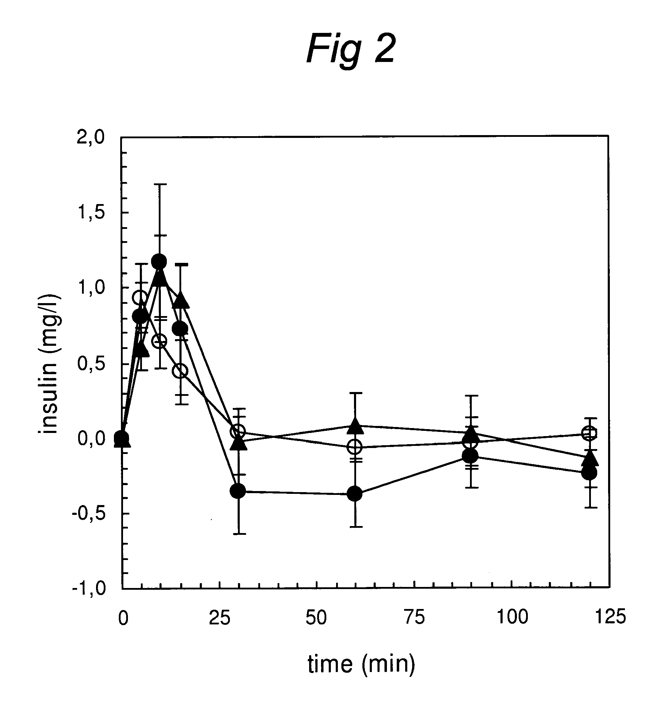

The post-prandial peak of glucose as well as of insulin is lower in rats fed intact whey and intact casein than in rats fed hydrolysed whey and hydrolysed casein. This can be seen in figure 1 and 2 and Table 1. The peak time of glucose is lower in rats fed hydrolysed whey and hydrolysed casein than in rats fed intact whey and intact casein. The area under the curve (AUC) of glucose is lower in rats fed hydrolysed whey and hydrolysed casein than in rats fed intact whey and intact casein. The presence of hydrolysed proteins resulted in post-prandial blood glucose as well as insulin levels and kinetics more similar to those observed with human milk (See figures).

Peak glucose times peak insulin and AUC glucose (0-30) times AUC insulin (0-30) are indications for insulin resistance. The lowest values were observed for rats fed human milk and rats fed hydrolysed casein and hydrolysed whey. The highest values were found for rats fed a mixture of non-hydrolysed whey and casein.

**Table 1: Effects of intact and hydrolysed proteins on post-prandial peak time, maximal peak height and area under the curve of glucose and insulin.**

| Effect | | Intact proteins | Hydrolysed proteins | Human milk |
|---|---|---|---|---|
| Peak time (m ± se) | | | | |
| | Glucose | 7.5 ± 3.8 | 11.5 ± 2.6 | 12.0 ± 2.4 |
| | Insulin | 8.3 ± 0.8 | 8.3 ± 1.4 | 11.7 ± 1.2 |
| Peak (± se) | | | | |
| | Glucose(g/l) | 0.46 ± 0.10 | 0.31 ± 0.07 | 0.33 ± 0.08 |
| | Insulin (mg/l) | 1.51 ± 0.44 | 1.11 ± 0.20 | 1.41 ± 0.27 |
| AUC 30 (± se) | | | | |
| | Glucose(min.g/l) | 5.3 ± 1.6 | 3.4 ± 2.1 | 3.6 ± 2.3 |
| | Insulin(min.mg/l) | 14.4 ± 7.7 | 12.5 ± 3.8 | 17.3 ± 4.3 |
| AUC 120 (± se) | | | | |
| | Glucose(min.g/l) | 8.1 ± 4.9 | -1.5 ± 9.0 | 3.5 ± 6.9 |
| | Insulin(min.mg/l) | -9.6 ± 23.2 | 10.6 ± 7.6 | 18.2 ± 21.3 |
| Peak glucose * peak insulin | | 5.9 ± 1.8 | 3.9 ± 0.6 | 4.5 ± 0.6 |
| AUCgluc*AUC insulin (0-30) | | 128 ± 78 | 81 ± 41 | 84 ± 53 |

### Example 2:

A powdered infant formula with a packaging indicating that the product is suitable for preventing childhood obesity or diseases following childhood obesity which can be reconstituted with water (15.6 g powder per 100 ml final volume) comprising per 100 ml ready to use product:

| | |
|---|---|
| Protein source¹: | 1.9 g 11 en. % |
| Fat: | 3.3 g 40 en. % |
| Digestible carbohydrates: | 8.7 g 49 en% |
| Non-digestible fermentable carbohydrate²: | 0.8 g |

| | |
|---|---|
| 1: Hydrolysed whey, hydrolysed casein in a weight ratio 6/4 as described in example 1 2: 0.72 g Transgalacto-oligosaccharide (present in Vivinal GOS, Borculo DOMO, The Netherlands) and 0.08 inulin (Raftlin HP, Orafti, Belgium). | |

## Claims

1. Use of fat, digestible carbohydrates and protein, wherein the protein comprises at least 25 wt. % peptides with a chain length of 2 to 30 amino acids based on dry weight of protein, and wherein the composition comprises at least 12 mg non-digestible fermentable carbohydrates per g dry weight of the nutritional composition, for the manufacture of a nutritional composition for use in the treatment and/or prevention of childhood obesity.

2. Use of fat, digestible carbohydrates and protein wherein the protein comprises at least 25 wt. % peptides with a chain length of 2 to 30 amino acids based on dry weight of protein, and wherein the composition comprises at least 12 mg non-digestible fermentable carbohydrates per g dry weight of the nutritional composition for the manufacture of a nutritional composition for use in the treatment and/or prevention of type 2 diabetes, asthma, arthritis, hypertension, sleep apnoea, cardiovascular diseases, and/or orthopaedic disorders in a subject suffering from childhood obesity.

3. Use according to claim 1 or 2 wherein the nutritional composition comprises at least 7 wt % protein based on dry weight of the nutritional composition.

4. Use according to any of the preceding claims, wherein the composition comprises hydrolysed whey protein and hydrolysed casein.

5. Use according to any of the preceding claims, wherein said nutritional composition is for oral administration to an infant.

6. Use according to any of the preceding claims wherein the nutritional composition comprises 5 to 16 en.% protein, 35 to 60 en.% fat, and 25 to 75 en.% carbohydrates.

7. Use according to any of the preceding claims wherein the protein comprises d) at least 50 wt. % mammalian milk derived proteins, based on weight of protein;
e) casein and whey in a weight ratio casein:whey of 10:90 to 90:10; and
f) less than 15 wt.% free amino acids based on the weight of the protein.

8. A nutritional composition comprising 35 to 60 en. % fat, 25 to 75 en. % digestible carbohydrates, 5 to 16 en. % protein, and a non-digestible fermentable carbohydrate selected from the group consisting of polyfructose and galactooligosaccarides, wherein the protein comprises
f) at least 25 wt. % peptides with a chain length of 2 to 30 amino acids based on dry weight of protein;
g) at least 50 wt. % mammalian milk derived proteins, based on weight of protein;
h) casein and whey in a weight ratio cascin:whey of 10:90 to 90:10;
i) hydrolysed whey protein and hydrolysed casein; and
j) less than 15 wt.% free amino acids based on the weight of protein source.

9. Use of the composition according to claim 8 for the manufacture of a composition for providing nutrition to an infant.

## Patentansprüche

1. Verwendung von Fett, verdaulichen Kohlenhydraten und Protein, worin das Protein, basierend auf dem Trockengewicht des Proteins, mindestens 25 Gew.-% Peptide mit einer Kettenlänge von 2 bis 30 Aminosäuren umfasst und worin die Zusammensetzung mindestens 12 mg unverdauliche, gärfähige Kohlenhydrate pro g Trockengewicht der Nahrungsmittelzusammensetzung umfasst, zur Herstellung einer Nahrungsmittelzusammensetzung zur Verwendung bei der Behandlung und/oder Verhütung von Fettleibigkeit im Kindesalter.

2. Verwendung von Fett, verdaulichen Kohlenhydraten und Protein, worin das Protein, basierend auf dem Trockengewicht des Proteins, mindestens 25 Gew.-% Peptide mit einer Kettenlänge von 2 bis 30 Aminosäuren umfasst und worin die Zusammensetzung mindestens 12 mg unverdauliche, gärfähige Kohlenhydrate pro g Trockengewicht der Nahrungsmittelzusammensetzung zur Herstellung einer Nahrungsmittelzusammensetzung umfasst, zur Verwendung bei der Behandlung und/oder Verhütung von Diabetes Typ 2, Asthma, Arthritis, Hypertonie, Schlafapnoe, kardiovaskulären Erkrankungen und/oder orthopädischen Störungen bei einem an Fettlebigkeit im Kindesalter leidenden Patienten.

3. Verwendung nach Anspruch 1 oder 2, worin die Nahrungsmittelzusammensetzung mindestens 7 Gew.-% Protein, basierend auf dem Trockengewicht der Nahrungsmittelzusammensetzung, umfasst.

4. Verwendung nach einem der vorhergehenden Ansprüche, worin die Zusammensetzung hydrolysiertes Molkeprotein und hydrolysiertes Kasein enthält.

5. Verwendung nach einem der vorhergehenden Ansprüche, worin die Nahrungsmittelzusammensetzung zur oralen Verabreichung an ein Säugling bestimmt ist.

6. Verwendung nach einem der vorhergehenden Ansprüche, worin die Nahrungsmittelzusammensetzung 5 bis 16 Energie-% Protein, 35 bis 60 Energie-% Fett und 25 bis 75 Energie-% Kohlenhydrate umfasst.

7. Verwendung nach einem der vorhergehenden Ansprüche, worin das Protein d) mindestens 50 Gew.-% aus der Milch eines Säugetiers stammende Proteine, basierend auf dem Gewicht des Proteins,
e) Kasein und Molke in einem Gewichtsverhältnis Kasein:Molke von 10:90 bis 90:10 und
f) weniger als 15 Gew.-% freie Aminosäuren, basierend auf dem Gewicht des Proteins,
umfasst.

8. Nahrungsmittelzusammensetzung umfassend 35 bis 60 Energie-% Fett, 25 bis 75 Energie-% verdauliche Kohlenhydrate, 5 bis 16 Energie-% Protein und ein aus der Gruppe bestehend aus Polyfructose und Galactooligosacchariden ausgewähltes, unverdauliches, gärfähiges Kohlenhydrat, worin das Protein f) mindestens 25 Gew.-% Peptide mit einer Kettenlänge von 2 bis 30 Aminosäuren, basierend auf dem Trockengewicht des Proteins,
g) mindestens 50 Gew.-%, basierend auf dem Gewicht des Proteins, aus der Milch eines Säugetiers stammende Proteine,
h) Kasein und Molke in einem Gewichtsverhältnis Kasein:Molke von 10:90 bis 90:10,
i) hydrolysiertes Molkeprotein und hydrolysiertes Kasein und
j) weniger als 15 Gew.-% freie Aminosäuren, basierend auf dem Gewicht der Proteinquelle,
umfasst.

9. Verwendung der Zusammensetzung nach Anspruch 8 zur Herstellung einer Zusammensetzung zur Bereitstellung von Säuglingsnahrung.

## Revendications

1. Utilisation de matière grasse, de glucides digestibles et de protéine, dans laquelle la protéine comprend au moins 25 % en poids de peptides ayant une longueur de chaîne de 2 à 30 acides aminés sur la base du poids sec de protéine, et dans laquelle la composition comprend au moins 12 mg de glucides fermentescibles non digestibles par g de poids sec de la composition nutritionnelle, pour la fabrication d'une composition nutritionnelle destinée à être utilisée dans le traitement et/ou la prévention de l'obésité infantile.

2. Utilisation de matière grasse, de glucides digestibles et de protéine, dans laquelle la protéine comprend au moins 25 % en poids de peptides ayant une longueur de chaîne de 2 à 30 acides aminés sur une base du poids sec de protéine, et dans laquelle la composition comprend au moins 12 mg de glucides fermentescibles non digestibles par g de poids sec de composition nutritionnelle, pour la fabrication d'une composition nutritionnelle destinée à être utilisée dans le traitement et/ou la prévention du diabète de type 2, de l'asthme, de l'arthrite, de l'hypertension, des apnées du sommeil, des maladies cardiovasculaires, et/ou des troubles orthopédiques chez un sujet souffrant d'obésité infantile.

3. Utilisation selon la revendication 1 ou 2, dans laquelle la composition nutritionnelle comprend au moins 7 % en poids de protéine sur la base du poids sec de la composition nutritionnelle.

4. Utilisation selon l'une quelconque des revendications précédentes, dans laquelle la composition comprend de la protéine lactosérique hydrolysée et de la caséine hydrolysée.

5. Utilisation selon l'une quelconque des revendications précédentes, dans laquelle ladite composition nutritionnelle est destinée à être administrée par voie orale à un nourrisson.

6. Utilisation selon l'une quelconque des revendications précédentes, dans laquelle la composition nutritionnelle comprend de 5 à 16 % d'énergie de protéine, de 35 à 60 % d'énergie de matière grasse, et de 25 à 75 % d'énergie de glucides.

7. Utilisation selon l'une quelconque des revendications précédentes, dans laquelle la protéine comprend :
d) au moins 50 % en poids de protéines dérivées de lait de mammifère, sur la base du poids de protéine ;
e) de la caséine et du lactosérum dans un rapport pondéral caséine:lactosérum allant de10:90à90:10;et
f) moins de 15 % en poids d'acides aminés libres sur la base du poids de protéine.

8. Composition nutritionnelle comprenant de 35 à 60 % d'énergie de matière grasse, de 25 à 75 % d'énergie de glucides digestibles, de 5 à 16 % d'énergie de protéine, et un glucide fermentescible non digestible choisi parmi le groupe constitué du polyfructose et des galacto-oligosaccharides, dans laquelle la protéine comprend :
f) au moins 25 % en poids de peptides ayant une longueur de chaîne de 2 à 30 acides aminés sur la base du poids sec de protéine ;
g) au moins 50 % en poids de protéines dérivées de lait de mammifère, sur la base du poids de protéine ;
h) de la caséine et du lactosérum dans un rapport pondéral caséine:lactosérum allant de 10:90 à 90:10 ;
i) de la protéine lactosérique hydrolysée et de la caséine hydrolysée ; et
j) moins de 15 % en poids d'acides aminés libres sur la base du poids de la source de protéine.

9. Utilisation de la composition selon la revendication 8, pour la fabrication d'une composition destinée à fournir une nutrition à un nourrisson.
